# EUROPEAN PATENT APPLICATION

(11) **EP 2 851 042 A1**
(43) Date of publication of application: **25.03.2015**
(21) Application number: 13185017.4
(22) Date of filing: 18.09.2013
(51) Int. Cl.: A61F 5/56

(54) **Medical implant system and the use thereof**

(71) Applicant: Rehasys AG, 4123 Allschwil (CH)
(72) Inventor: The designation of the inventor has not yet been filed
(74) Representative: Braitmayer, Sven-Erik

(57) **Abstract**

The present invention relates to a medical implant system (100; 100'; 100"; 100"') for the implantation in the tracheal region, especially for the treatment of sleep apnea, wherein the implant system (100; 100'; 100''; 100''') comprises at least two magnetic means (10; 10'; 10''; 10'''), wherein the magnetic means (10; 10'; 10"; 10"') are configured and arranged such that the tracheal region at the implantation site remains open even in a relaxed state of the muscles around the tracheal region and/or during breathing in due to an at least partial repelling effect between the at least two magnetic means (10; 10'; 10''; 10''').

Furthermore, the present invention relates to use of a medical implant system (100; 100'; 100''; 100''') for the treatment of sleep apnea.

## Description

The present invention relates to a medical implant system, in particular to a medical implant system for the treatment of sleep apnea and also to the use of a medical implant system for the treatment of sleep apnea.

Sleep apnea is a type of sleep disorder characterized by pauses in breathing or instances of shallow or infrequent breathing during sleep. Each pause in breathing, called an apnea, can last from at least ten seconds to minutes, and may occur 5 to 30 times or more an hour. Similarly, each abnormally shallow breathing event is called a hypopnea. Sleep apnea is often diagnosed with an overnight sleep test called a polysomnogram, or "sleep study".

There are three forms of sleep apnea: central (CSA), obstructive (OSA), and complex or mixed sleep apnea (i.e., a combination of central and obstructive) constituting 0.4%, 84% and 15% of cases respectively. In CSA, breathing is interrupted by a lack of respiratory effort; in OSA, breathing is interrupted by a physical block to airflow despite respiratory effort, and snoring is common.

Regardless of type, an individual with sleep apnea is rarely aware of having difficulty breathing, even upon awakening. Sleep apnea is recognized as a problem by others witnessing the individual during episodes or is suspected because of its effects on the body (sequelae). Symptoms may be present for years (or even decades) without identification, during which time the sufferer may become conditioned to the daytime sleepiness and fatigue associated with significant levels of sleep disturbance.

Obstructive sleep apnea (OSA) is the most common category of sleep-disordered breathing. The muscle tone of the body ordinarily relaxes during sleep, and at the level of the throat the human airway is composed of collapsible walls of soft tissue which can obstruct breathing during sleep. Mild occasional sleep apnea, such as many people experience during an upper respiratory infection, may not be important, but chronic severe obstructive sleep apnea requires treatment to prevent low blood oxygen (hypoxemia), sleep deprivation, and other complications.

Individuals with low muscle tone and soft tissue around the airway (e.g., because of obesity) and structural features that give rise to a narrowed airway are at high risk for obstructive sleep apnea. The elderly are more likely to have OSA than young people. Men are more likely to suffer sleep apnea than women and children are, though it is not uncommon in the last two population groups.

The risk of OSA rises with increasing body weight, active smoking and age. In addition, patients with diabetes or "borderline" diabetes have up to three times the risk of having OSA.

Common symptoms include loud snoring, restless sleep, and sleepiness during the daytime. Diagnostic tests include home oximetry or polysomnography in a sleep clinic.

Some treatments involve lifestyle changes, such as avoiding alcohol or muscle relaxants, losing weight, and quitting smoking. Many people benefit from sleeping at a 30-degree elevation of the upper body or higher, as if in a recliner. Doing so helps prevent the gravitational collapse of the airway. Lateral positions (sleeping on a side), as opposed to supine positions (sleeping on the back), are also recommended as a treatment for sleep apnea, largely because the gravitational component is smaller in the lateral position. Some people benefit from various kinds of oral appliances to keep the airway open during sleep. Continuous positive airway pressure (CPAP) is the most effective treatment for severe obstructive sleep apnea but oral appliances are considered a first line approach equal to CPAP for mild to moderate sleep apnea according to the AASM parameters of care. There are also surgical procedures to remove and tighten tissue and widen the airway.

All above known treatment approaches are at least of minor convenience for the patient and it would be desireable to have a more effective treatment option for the patients, which is also easy to apply.

It is therefore an object of the present invention to improve an implant system and the use of an implant system for the treatment of sleep apnea as mentioned above, in particular in that the implant system and its use allows a more effective treatment and which is also easy to implant and/or apply to a patient.

This object is solved according to the present invention by a medical implant system with the features of claim 1. Accordingly, a medical implant system for the implantation in the tracheal region is provided, especially for the treatment of sleep apnea, wherein the implant system comprises at least two magnetic means, wherein the magnetic means are configured and arranged such that the tracheal region at the implantation site remains open even in a relaxed state of the muscles around the tracheal region and/or during breathing in due to an at least partial repelling effect between the at least two magnetic means.

By this, the advantage is achieved that such an implant system according to the present invention allows a more effective treatment and is also easy to implant and/or apply to a patient. In particular, e.g. no CPAP-system has to be used by the patients during the night which is a large improvement of quality of life. Further, the surgical procedure needed for the implantation can be done minimally invasive and with low risk for the patient. The implant system can be designed with small dimensions and will be still effective to keep the airways normally being obstructed during sleep apnea open. So, no big incisions are needed and thus the recovery of the patient after the implantation is very fast. Furthermore, the implantation system is immediately operable after implantation.

For example, two or more magnetic means can be e.g. minimally invasively implanted and arranged such that they are repelling each other due to their magnetic characteristics. It is also possible that the magnetic means are configured and arranged such that an additional magnetic field attracts the magnetic means such that the obstructed airway during sleep apnea will remain open.

It is possible that the length of the magnetic means is for example chosen in a range of 0.1 mm to 10 mm. Generally, all suitable lengths may be chosen.

Also, the diameter of the magnetic means is for example chosen in a range of 0.1 mm to 10 mm. Generally, all suitable diameters may be chosen.

Moreover, it is possible that the magnetic means comprise each at least one position securing and/or fixing means configured and arranged to secure and/or fix the position of the magnetic means at the implantation side. By means of the position securing and/or fixing means it is advantageously possible to prevent a change of orientation of the implanted implant system and/or the magnetic means.

Also, it is possible that the position securing and/or fixing means are or comprise a hook and/or a clamp and/or plate or the like. Such structures are easily to realize and very effective to prevent a change of orientation of the implanted magnetic means.

Additionally, it is possible that the magnetic means are at least partially coated with a biocompatible material, wherein for example the biocompatible material is a biocompatible polymer and/or wherein for example the magnetic means are completely coated. The use of a biocompatible material helps to prevent inflammation reactions and ensures that the implant system causes no complications after implantation.

Also, it is possible that the implant system and/or at least one of the magnetic means has an at least partially tubular shape, wherein the shape is arranged such that the tracheal region is at least partially coverable. A tubular shape can be easily implanted and can also immediately ensure that there will be no change in the orientation of the implant after implantation.

Moreover, it is possible that the implant system and/or at least one of the magnetic means has an at least partially a stent-like structure and/or shape, wherein for example the stent-like structure is at least partially folded in the pre-implanted state and at least partially unfolded in the implanted state. By this, the advantage can be achieved that in the partially folded state the magnetic means can be introduces into the tracheal region through the mouth and/or nose without any incision. So, the recovery of the patient is very fast since there are no incisions that must be sutured and there are no open wounds.

Furthermore, it is possible that the implant system and/or at least one of the magnetic means has at least partially a net structure and/or a lattice structure. Such a structure allows less irritations when placed in the tracheal area since the outer face being in contact with the surrounding tissue is reduced and at the same time this structure still allows more than a sufficient stability.

In particular, it is possible that the implant system and/or at least one of the magnetic means is at least partially implantable through the mouth to an implantation site adjacent and/or next to and/or inside of the throat, preferably by means of a minimally invasive surgical procedure.

Additionally, it is possible that the implant system and/or at least one of the magnetic means is at least partially implantable through at least one incision of the neck outside of and/or adjacent and/or next to the throat.

Also, it is possible that the implant system and/or at least one of the magnetic means is configured such that it comprises at least a first folded state in which the implant system and/or at least one of the magnetic means has a cross-section which allows a transport through an implantation cannula and at least a second unfolded state with a larger cross-section being larger than the cross-section of the cannula. The implantation by means of a cannula allows a first time correct placement and thus helps to keep the necessary implantation wounds as low as possible.

Further, it is possible that the implant system comprises at least one magnetic field generating means which is configured and arranged such that the magnetic field generation means is arranged outside of the body of a patient and capable to attract the implanted magnetic means inside of the body of the patient to a direction towards the magnetic field generating means. For example, it is possible that the patient may use the exterior magnetic field generation means while sleeping. The use of an exterior magnetic field generation means reduces the complexity of the implantation procedure since the orientation of magnetic means is irrelevant. This allows an easier implantation of the magnetic means.

Additionally, the implant system and/or at least one of the magnetic means comprises a cross-section with a height and a width, wherein height and width are chosen such that they are not equal, wherein exemplarily the cross-section is rectangular with rounded edges or oval or free-formed with rounded edges. Such a cross-section helps to maintain the orientation of the implant system and/or at least one of the magnetic means after implantation, since e.g. no rotation along the axial axis is possible. This can be e.g. achieved by provided a larger height than width or by providing a larger width than height when designing the cross-section.

It is possible that the implant system and/or at least one of the magnetic means has/have at their outer face only rounded edges. Rounded edges and round forms help to prevent inflammation caused by an implant at the implantation site.

Moreover, it is possible that the implant system and/or at least one of the magnetic means consist(s) at least partially of or is at least partially covered and/or coated by a shape-memory material, wherein exemplarily the shape-memory material is a shape-memory polymer or a shape-memory metal or metal alloy.

Shape-memory polymers (SMPs) for example are polymeric smart materials that have the ability to return from a deformed state (temporary shape) to their original (permanent) shape induced by an external stimulus (trigger), such as temperature change. It is possible to use such an SMP and its effect to unfold the medical implant system and/or the magnetic means after implantation into the body of the patient, for example to prevent a rotation or any other change of the orientation of the implant. In particular, it is possible to use the body temperature of the patient to trigger the shape-memory effect for unfolding the implant system and/or the magnetic means.

Examples of such SMPs are physically crosslinked SMPs or chemically crosslinked SMPs.

Physically crosslinked SMPs are for example linear block copolymers or other thermoplastic polymers. Representative shape-memory polymers in the category of linear block copolymers are polyurethanes, polyurethanes with ionic or mesogenic components made by prepolymer method. Other block copolymers also show the shape-memory effect, such as, block copolymer of polyethylene terephthalate (PET) and polyethyleneoxide (PEO), block copolymers containing polystyrene and poly(1,4-butadiene), and an ABA triblock copolymer made from poly(2-methyl-2-oxazoline) and polytetrahydrofuran. Other thermoplastic polymers are for example linear, amorphous polynorbornene (Norsorex, developed by CdF Chemie/Nippon Zeon) or organic-inorganic hybrid polymers consisting of polynorbornene units that are partially substituted by polyhedral oligosilsesquioxane (POSS) also have shape-memory effect.

Examples for chemically crosslinked SMPs are crosslinked polyurethane and PEO based crosslinked SMPs. Crosslinked polyurethane can be made by using excess diisocyanate or by using a crosslinker such as glycerin, trimethylol propane. Introduction of covalent crosslinking improves in creep, increase in recovery temperature and recovery window. The PEO-PET block copolymers can be crosslinked by using maleic anhydride, glycerin or dimethyl 5-isopthalates as a crosslinking agent. The addition of 1.5 wt% maleic anhydride increased in shape recovery from 35% to 65% and tensile strength from 3 to 5 MPa.

Furthermore, the present invention relates to the use of a medical implant system for the treatment of sleep apnea. Accordingly, a medical implant system according to one of claims 1 to 14 for the treatment of sleep apnea is used.

By this, the advantage is achieved that such a use of an implant system according to the present invention allows a more effective treatment and also easy to implant and/or apply to a patient. In particular, e.g. no CPAP-system has to be used by the patients during the night which is a large improvement of quality of life. Further, the surgical procedure needed for the implantation can be done minimally invasive and with low risk for the patient. No big incisions are needed and thus the recovery of the patient after the implantation is very fast. Furthermore, the implantation system is immediately operable after implantation.

Further advantages and details of the present invention shall be described hereinafter in connection with an exemplary embodiment of the present invention shown in the drawings. In the drawings it is shown:
- Fig. 1 a side sectional view of head of a patient suffering sleep apnea with obstructed airways;
- Fig. 2 a side sectional view of head of a patient with an implanted medical implant system according to the present invention in a first embodiment and its use according to the present invention to treat sleep apnea;
- Fig. 3 a side sectional view of head of a patient with an implanted medical implant system according to the present invention in a second embodiment and its use according to the present invention to treat sleep apnea;
- Fig. 4 a side sectional view of head of a patient with an implanted medical implant system according to the present invention in a third embodiment and its use according to the present invention to treat sleep apnea;
- Fig. 5 a side sectional view of head of a patient with an implanted medical implant system according to the present invention in a fourth embodiment and its use according to the present invention to treat sleep apnea; and
- Fig. 6 a further side sectional view of head of a patient with an implanted medical implant system according to the present invention in a fourth embodiment and its use according to the present invention to treat sleep apnea together with an external magnetic field generator.

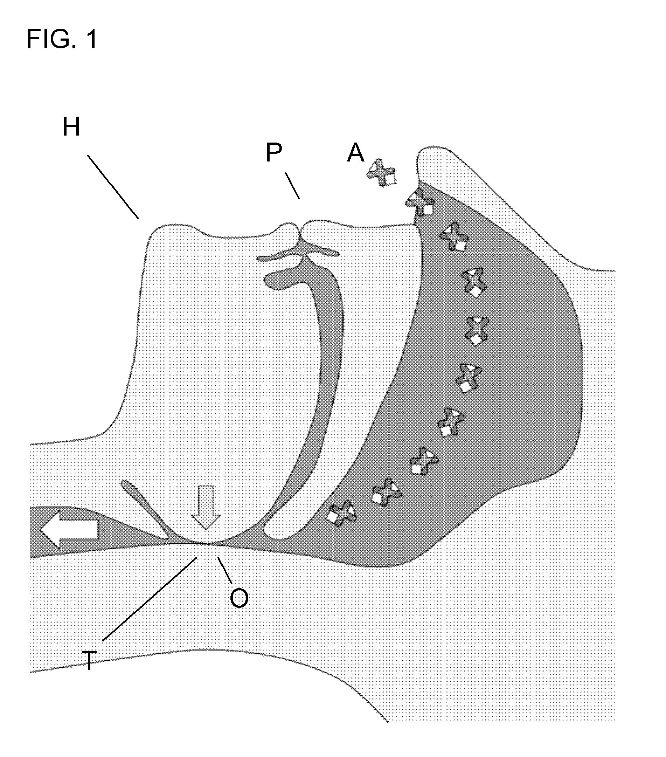

Figure 1 shows side sectional view of head H of a patient P suffering sleep apnea with obstructed airways. The obstruction O is in the tracheal region T prevents air A from entering the airways.

The shown side sectional view is an example for obstructive sleep apnea (OSA), which is the most common category of sleep-disordered breathing. The muscle tone of the body ordinarily relaxes during sleep, and at the level of the throat the human airway is composed of collapsible walls of soft tissue which can obstruct breathing during sleep as shown in Figure 1.

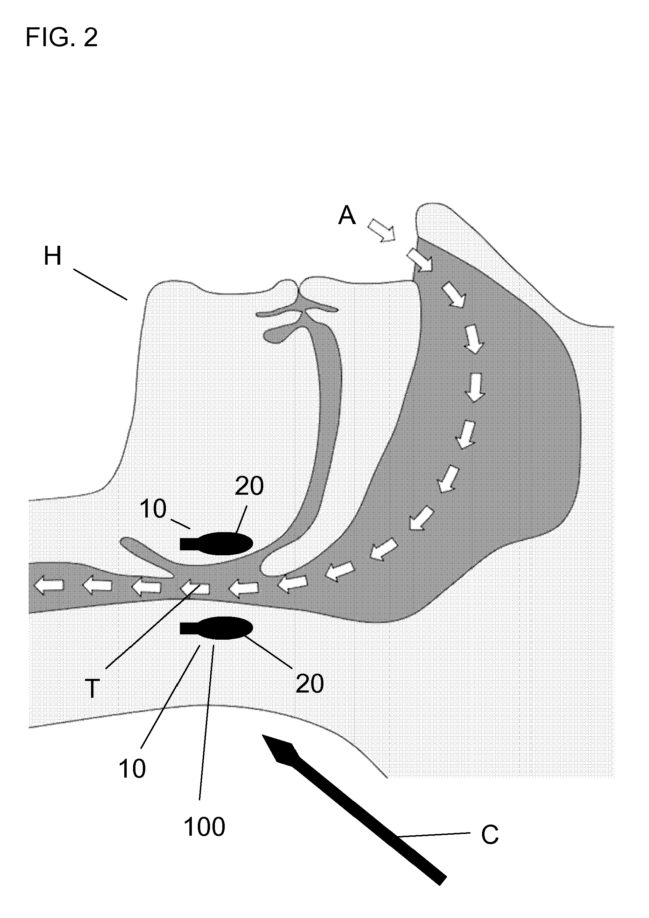

Figure 2 shows a side sectional view of head H of a patient P with an implanted medical implant system 100 according to the present invention and its use according to the present invention to treat sleep apnea.

The medical implant system 100 is implanted minimally invasive by means of an implantation cannula C in the tracheal region T for the treatment of sleep apnea.

The implant system 100 comprises here two magnetic means 10, wherein the magnetic means 10 are configured and arranged such that the tracheal region at the implantation site remains open even in a relaxed state of the muscles around the tracheal region T and during breathing in due to an at least partial repelling effect between the at least two magnetic means 10.

The magnetic means 10 comprise each at least one position securing and fixing means 20 configured and arranged to secure and/or fix the position of the magnetic means 10 at the implantation side. By means of the position securing and/or fixing means it is advantageously possible to prevent a change of orientation of the magnetic means and so a correct function of the implant system can be ensured.

The magnetic means 10 are completely coated with a biocompatible polymer such as biocompatible polyurethane or other suitable biocompatible polymers. The use of a biocompatible material helps to prevent inflammation reactions and ensures that the implant system 100 causes no complications after implantation.

The magnetic means 10 comprise a cross-section with a height and a width, wherein height and width are chosen such that they are not equal, wherein the cross-section is rectangular with rounded edges. This cross-section helps to maintain the orientation of the implant system and/or at least one of the magnetic means after implantation, since e.g. no rotation along the axial axis is possible.

The shown embodiment of the implant system 100 and its magnetic means 10 is at least partially implantable through at least one incision of the neck outside of and/or adjacent and/or next to the throat via an implantation cannula C.

The implant system 100 and the magnetic means 10 may consist at least partially of a shape-memory material, wherein exemplarily the shape-memory material is a shape-memory polymer or a shape-memory metal or metal alloy. This helps to unfold the position securing and fixing means 20 after implantation.

The position securing and/or fixing means 20 can comprise a hook and/or a clamp and/or plate or the like and is here embodied as an oval plate.

Also, the magnetic means 10 may have at least partially a tubular shape, wherein the shape is arranged such that the tracheal region T is at least partially coverable. The tubular shape may be e.g. a tube with a slit.

The embodiment shown in Figure 2 comprises for example the advantage that it allows a more effective treatment and is also easy to implant and/or apply to a patient. In particular, e.g. no CPAP-system has to be used by the patients during the night which is a large improvement of quality of life. Further, the surgical procedure needed for the implantation can be done minimally invasive and with low risk for the patient. The implant system 100 is designed with small dimensions and will be still effective to keep the airways normally being obstructed during sleep apnea open. So, no big incisions are needed and thus the recovery of the patient P after the implantation is very fast. Furthermore, the implantation system 100 is immediately operable after implantation.

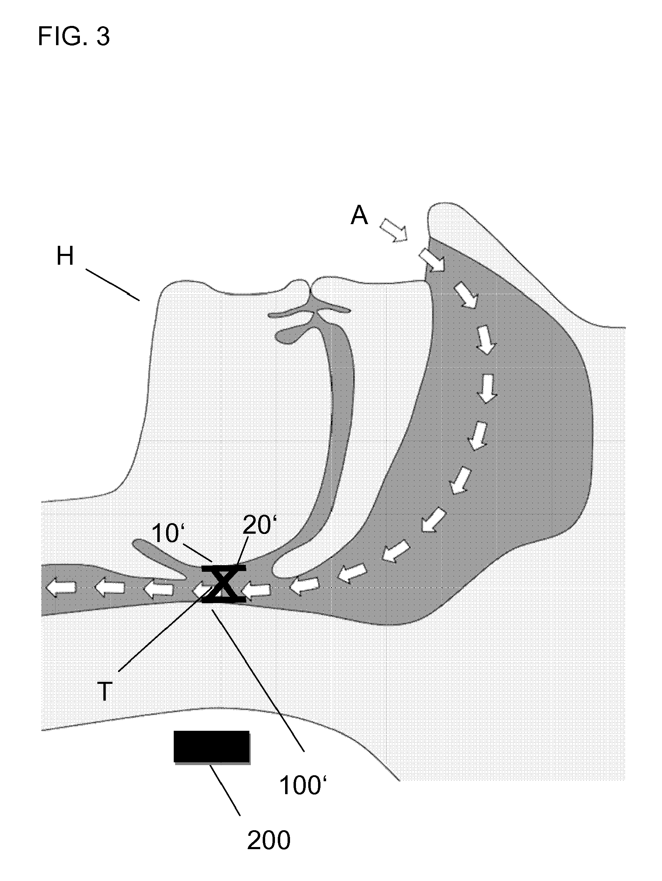

Figure 3 shows a side sectional view of head H of a patient P with an implanted medical implant system 100' according to the present invention and its use according to the present invention to treat sleep apnea.

The medical implant system 100' with its magnetic means 10 has a tubular shape, wherein the shape is arranged such that the tracheal region is at least partially coverable. The magnetic means 10 are part of the implant system and covered by a biocompatible coating.

The implant system 100' has a stent-like structure and shape, wherein for example the stent-like structure is at least partially folded in the pre-implanted state and at least partially unfolded in the implanted state. Further, the implant system 100' and the magnetic means 10' have an at least partially a net structure and/or a lattice structure. The position securing and/or fixing means 20 can comprise a hook and/or a clamp and/or plate or the like and is here embodied as a small hooks.

The implant system 100 can be implanted through the mouth to an implantation site adjacent and/or next to and/or inside of the throat, preferably by means of a minimally invasive surgical procedure.

To support the effect of the implant system there can be a field generating means 200 which is configured and arranged such that the magnetic field generation means is arranged outside of the body of a patient and capable to attract the implanted magnetic means 10 inside of the body of the patient to a direction towards the magnetic field generating means 200.

The implant system 100' and the magnetic means 10 may consist at least partially of a shape-memory material, wherein exemplarily the shape-memory material is a shape-memory polymer or a shape-memory metal or metal alloy. This helps to unfold the position securing and fixing means 20 after implantation.

The embodiment shown in Figure 3 comprises for example the advantage that it allows a more effective treatment and is also easy to implant and/or apply to a patient. In particular, e.g. no CPAP-system has to be used by the patients during the night which is a large improvement of quality of life. Further, the surgical procedure needed for the implantation can be done minimally invasive and with low risk for the patient. The implant system 100' is designed with small dimensions and will be still effective to keep the airways normally being obstructed during sleep apnea open. So, no big incisions are needed and thus the recovery of the patient P after the implantation is very fast. Furthermore, the implantation system 100' is immediately operable after implantation.

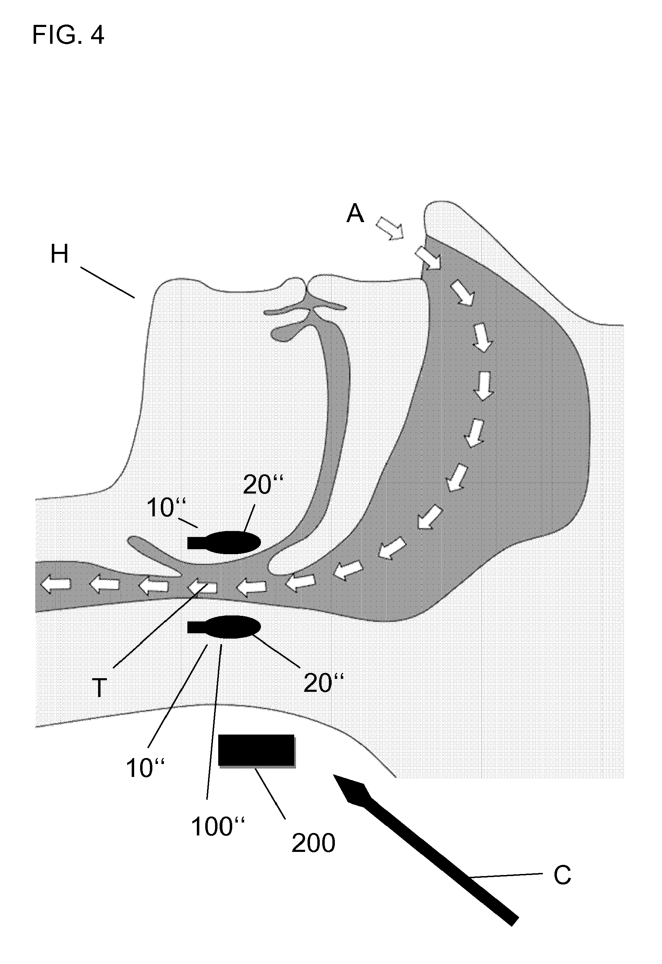

Figure 4 shows a side sectional view of head H of a patient P with an implanted medical implant system 100" (third example of a possible embodiment) according to the present invention and its use according to the present invention to treat sleep apnea.

Likewise the embodiment shown in Figure 2, the medical implant system 100" is implanted minimally invasive by means of an implantation cannula C in the tracheal region T for the treatment of sleep apnea.

The implant system 100" comprises here two (or more) magnetic means 10", wherein the magnetic means 10" are configured and arranged such that the tracheal region at the implantation site remains open even in a relaxed state of the muscles around the tracheal region T and during breathing in due to an at least partial repelling effect between the at least two magnetic means 10".

To support the effect of the implant system 100" there is a field generating means 200 which is configured and arranged such that the magnetic field generation means is arranged outside of the body of a patient and capable to attract the implanted magnetic means 10" inside of the body of the patient to a direction towards the magnetic field generating means 200.

The magnetic means 10" comprise each at least one position securing and fixing means 20" configured and arranged to secure and/or fix the position of the magnetic means 10" at the implantation side. By means of the position securing and/or fixing means it is advantageously possible to prevent a change of orientation of the magnetic means and so a correct function of the implant system can be ensured.

The magnetic means 10" are completely coated with a biocompatible polymer such as biocompatible polyurethane or other suitable biocompatible polymers. The use of a biocompatible material helps to prevent inflammation reactions and ensures that the implant system 100 causes no complications after implantation.

The magnetic means 10" comprise a cross-section with a height and a width, wherein height and width are chosen such that they are not equal, wherein the cross-section is rectangular with rounded edges. This cross-section helps to maintain the orientation of the implant system and/or at least one of the magnetic means after implantation, since e.g. no rotation along the axial axis is possible.

The shown embodiment of the implant system 100" and its magnetic means 10" is at least partially implantable through at least one incision of the neck outside of and/or adjacent and/or next to the throat via an implantation cannula C.

The implant system 100" and the magnetic means 10" may consist at least partially of a shape-memory material, wherein exemplarily the shape-memory material is a shape-memory polymer or a shape-memory metal or metal alloy. This helps to unfold the position securing and fixing means 20" after implantation.

The position securing and/or fixing means 20" can comprise a hook and/or a clamp and/or plate or the like and is here embodied as a oval plate.

The embodiment shown in Figure 4 comprises for example the advantage that it allows a more effective treatment and is also easy to implant and/or apply to a patient. In particular, e.g. no CPAP-system has to be used by the patients during the night which is a large improvement of quality of life. Further, the surgical procedure needed for the implantation can be done minimally invasive and with low risk for the patient. The implant system 100" is designed with small dimensions and will be still effective to keep the airways normally being obstructed during sleep apnea open. So, no big incisions are needed and thus the recovery of the patient P after the implantation is very fast. Furthermore, the implantation system 100" is immediately operable after implantation.

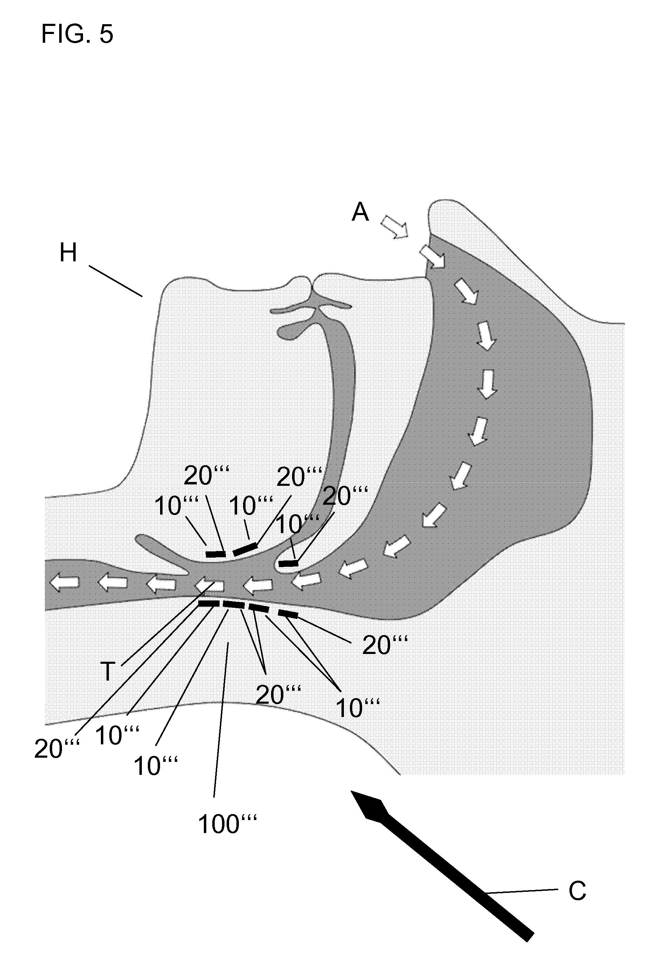

Figure 5 shows a side sectional view of head H of a patient P with a fourth embodiment of an implanted medical implant system 100"' according to the present invention and its use according to the present invention to treat sleep apnea.

Likewise the embodiment shown in Figure 2, the medical implant system 100"' is implanted minimally invasive by means of an implantation cannula C in the tracheal region T for the treatment of sleep apnea.

The implant system 100"' comprises here several magnetic means 10"', wherein the magnetic means 10"' are configured and arranged such that the tracheal region at the implantation site remains open even in a relaxed state of the muscles around the tracheal region T and during breathing in due to an at least partial repelling effect between the at least two magnetic means 10"'.

There is at least one magnetic means 10"', which is implanted in the soft palate region (and there may be more around this region) and there are magnetic means 10"' implanted in and around the back part of the tongue and there are several magnetic means 10"' implanted around in the pharynx region.

The magnetic means 10"' are smaller than the magnetic means 10 as shown in Figure 2 and comprise each at least one position securing and fixing means 20"' configured and arranged to secure and/or fix the position of the magnetic means 10"' at the implantation side. By means of the position securing and/or fixing means it is advantageously possible to prevent a change of orientation of the magnetic means and so a correct function of the implant system can be ensured.

All magnetic means 10"' are preferably identical, but it is also possible that there are magnetic means 10"' of different size.

The magnetic means 10"' are completely coated with a biocompatible polymer such as biocompatible polyurethane or other suitable biocompatible polymers. The use of a biocompatible material helps to prevent inflammation reactions and ensures that the implant system 100 causes no complications after implantation.

The magnetic means 10"' comprise a cross-section with a height and a width, wherein height and width are chosen such that they are not equal, wherein the cross-section is rectangular with rounded edges. This cross-section helps to maintain the orientation of the implant system and/or at least one of the magnetic means after implantation, since e.g. no rotation along the axial axis is possible.

The shown embodiment of the implant system 100"' and its magnetic means 10"' is at least partially implantable through at least one incision of the neck outside of and/or adjacent and/or next to the throat via an implantation cannula C.

The implant system 100"' and the magnetic means 10"' may consist at least partially of a shape-memory material, wherein exemplarily the shape-memory material is a shape-memory polymer or a shape-memory metal or metal alloy. This helps to unfold the position securing and fixing means 20"' after implantation.

The position securing and/or fixing means 20"' can comprise a hook and/or a clamp and/or plate or the like and is embodied as an oval plate.

The embodiment shown in Figure 5 comprises for example the advantage that it allows a more effective treatment and is also easy to implant and/or apply to a patient. In particular, e.g. no CPAP-system has to be used by the patients during the night which is a large improvement of quality of life. Further, the surgical procedure needed for the implantation can be done minimally invasive and with low risk for the patient. The implant system 100"' is designed with small dimensions and will be still effective to keep the airways normally being obstructed during sleep apnea open. So, no big incisions are needed and thus the recovery of the patient P after the implantation is very fast. Furthermore, the implantation system 100"' is immediately operable after implantation.

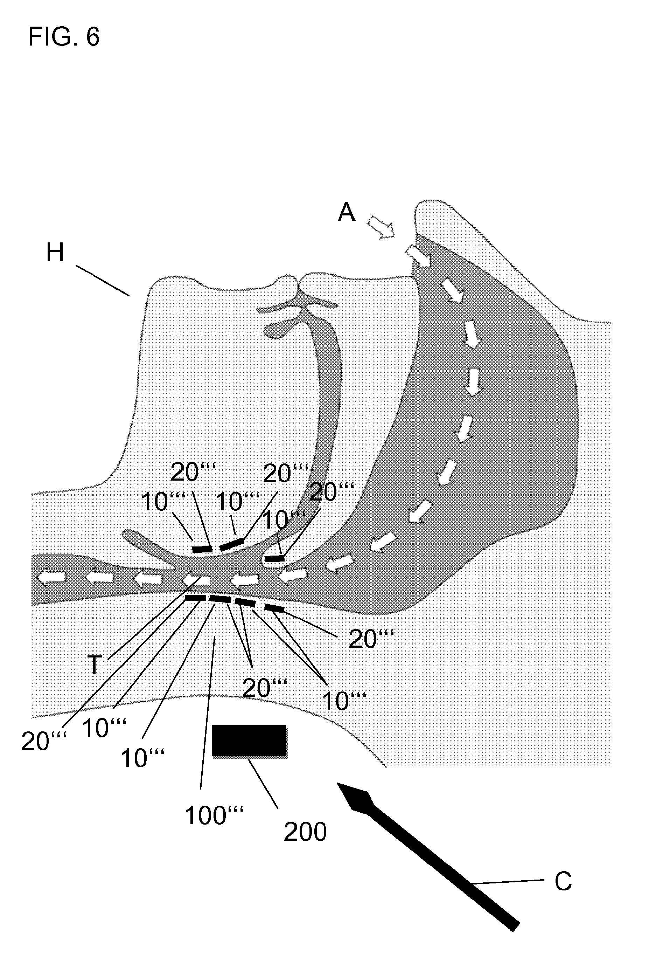

As shown in Figure 6, it is possible that for supporting the effect of the implant system 100"' as shown in Figure 5 there is a field generating means 200 which is configured and arranged such that the magnetic field generation means is arranged outside of the body of a patient and capable to attract the implanted magnetic means 10"' inside of the body of the patient to a direction towards the magnetic field generating means 200.

## Claims

1. A medical implant system (100; 100'; 100"; 100"') for the implantation in the tracheal region (T), especially for the treatment of sleep apnea, wherein the implant system (100; 100'; 100"; 100"') comprises at least two magnetic means (10; 10'; 10"; 10"'), wherein the magnetic means (10; 10'; 10"; 10"') are configured and arranged such that the tracheal region (T) at the implantation site remains open even in a relaxed state of the muscles around the tracheal region (T) and/or during breathing in due to an at least partial repelling effect between the at least two magnetic means (10; 10'; 10"; 10"').

2. The medical implant system (100; 100'; 100"; 100"') according to claim 1, **characterized in that** the magnetic means (10; 10'; 10"; 10"') comprise each at least one position securing and/or fixing means (20; 20'; 20"; 20"') configured and arranged to secure and/or fix the position of the magnetic means (10; 10'; 10"; 10"') at the implantation side.

3. The medical implant system (100; 100'; 100"; 100"') according to claim 2, **characterized in that** the position securing and/or fixing means (20; 20'; 20"; 20"') are or comprise a hook and/or a clamp and/or plate or the like.

4. The medical implant system (100; 100'; 100"; 100"') according to one of the preceding claims, **characterized in that** the magnetic means (10; 10'; 10"; 10"') are at least partially coated with a biocompatible material, wherein for example the biocompatible material is a biocompatible polymer and/or wherein for example the magnetic means (10; 10'; 10"; 10"') are completely coated.

5. The medical implant system (100; 100'; 100"; 100"') according to one of the preceding claims, **characterized in that** the implant system (100; 100'; 100"; 100"') and/or at least one of the magnetic means (10; 10'; 10"; 10"') has at least partially a tubular shape, wherein the shape is arranged such that the tracheal region is at least partially coverable.

6. The medical implant system (100') according to one of the preceding claims, **characterized in that** the implant system (100') and/or at least one of the magnetic means (10') has at least partially a stent-like structure and/or shape, wherein for example the stent-like structure is at least partially folded in the pre-implanted state and at least partially unfolded in the implanted state.

7. The medical implant system (100') according to one of the preceding claims, **characterized in that** the implant system (100') and/or at least one of the magnetic means (10') has an at least partially a net structure and/or a lattice structure.

8. The medical implant system (100') according to one of the preceding claims, **characterized in that** the implant system (100') and/or at least one of the magnetic means (10') is at least partially implantable through the mouth to an implantation site adjacent and/or next to and/or inside of the throat, preferably by means of a minimally invasive surgical procedure.

9. The medical implant system (100; 100'; 100") according to one of the preceding claims, **characterized in that** the implant system (100; 100'; 100") and/or at least one of the magnetic means (10') is at least partially implantable through at least one incision of the neck outside of and/or adjacent and/or next to the throat.

10. The medical implant system (100; 100"; 100"') according to one of the preceding claims, **characterized in that** the implant system (100; 100"; 100"') and/or at least one of the magnetic means (10; 10"; 10"') is configured such that it comprises at least a first folded state in which the implant system (100; 100'; 100") and/or at least one of the magnetic means (10; 10"; 10"') has a cross-section which allows a transport through an implantation cannula and at least a second unfolded state with a larger cross-section being larger than the cross-section of the cannula (C).

11. The medical implant system (100'; 100"; 100"') according to one of the preceding claims, **characterized in that** the implant system (100'; 100"; 100"') comprises at least one magnetic field generating means (200) which is configured and arranged such that the magnetic field generation means (200) is arranged outside of the body of a patient (P) and capable to attract the implanted magnetic means (10'; 10"; 10"') inside of the body of the patient to a direction towards the magnetic field generating means (200).

12. The medical implant system (100'; 100"; 100"') according to one of the preceding claims, **characterized in that** the implant system (100'; 100"; 100"') and/or at least one of the magnetic means (10'; 10"; 10"') comprises a cross-section with a height and a width, wherein height and width are chosen such that they are not equal, wherein exemplarily the cross-section is rectangular with rounded edges or oval or free-formed with rounded edges.

13. The medical implant system (100; 100'; 100"; 100"') according to one of the preceding claims, **characterized in that** the implant system (100; 100'; 100"; 100"') and/or at least one of the magnetic means (10; 10'; 10"; 10"') has/have at their outer face only rounded edges.

14. The medical implant system (100; 100'; 100"; 100"') according to one of the preceding claims, **characterized in that** the implant system (100; 100'; 100"; 100"') and/or at least one of the magnetic means (10; 10'; 10"; 10"') consist(s) at least partially of or is at least partially covered and/or coated by a shape-memory material, wherein exemplarily the shape-memory material is a shape-memory polymer or a shape-memory metal or metal alloy.

15. The use of a medical implant system (100; 100'; 100"; 100"') according to one of claims 1 to 14 for the treatment of sleep apnea.
